# EUROPEAN PATENT SPECIFICATION

(11) **EP 1 509 227 B1**
(45) Date of publication and mention of the grant of the patent: **07.10.2009**
(21) Application number: 03756140.4
(22) Date of filing: 30.05.2003
(51) Int. Cl.: A61K 31/465, A61K 9/08, A61K 9/12, A61K 9/72, A61P 25/34

(54) **A BUFFERED, LIQUID NICOTINE COMPOSITION FOR PULMONARY ADMINISTRATION**
GEPUFFERTE FLÜSSIGE NIKOTINZUSAMMENSETZUNG ZUR PULMONALEN VERABREICHUNG
COMPOSITION NICOTINIQUE LIQUIDE TAMPONNEE POUR ADMINISTRATION DANS LES POUMONS

(30) Priority: 03.06.2002 SE 0201669
(43) Date of publication of application: 02.03.2005
(73) Proprietor: McNeil AB, 25109 Helsingborg (SE)
(72) Inventor: WARCHOL, Mark, P. Pfizer Global Research and Dev., 267-633 Kalamazoo, MI 49001 (US); MOREN, Folke, S-216 18 Limhamn (SE); THYRESSON, Kristina Pfizer Health AB, S-251 09 Helsingborg (SE); STHENGEL, Elisabeth Pfizer Health AB, S-251 09 Helsingborg (SE); ANDERSSON, Sven-Börje Pfizer Health AB, S-251 09 Helsingborg (SE)
(74) Representative: Hedenström, John Allan
(86) International application number: PCT/SE2003/000890
(87) International publication number: WO 2003/101454

(56) References cited:
- DE-A1- 3 241 437
- GB-A- 2 030 862
- US-A- 4 579 858
- US-A- 4 920 989
- US-A- 4 953 572
- US-A- 5 135 753
- US-A- 5 656 255

## Description

### Technical Field

The present invention relates to a liquid pharmaceutical formulation for delivering nicotine to a subject by inhalation so that administration of the nicotine is to the lungs. This invention also relates to use of said liquid pharmaceutical formulation.

### Background of the Invention

### Tobacco dependence and reduction thereof

In recent years, with the recognition of the harmful effects of tobacco smoking, there have been numerous campaigns and programs by governmental agencies and various health groups and other interested organizations to disseminate information about the adverse health effects resulting from tobacco smoking. Moreover, and as a result of this recognition of the harmful effects, there have been many programs directed to attempts in reducing smoking incidence.

Nicotine is an organic compound and is the principal alkaloid of tobacco. Nicotine is the chief active ingredient in the tobacco used in cigarettes, cigars, snuff and the like. Nicotine is also an addictive drug, though, and smokers characteristically display a strong tendency to relapse after having successfully stopped smoking for a time. Nicotine is the world's second most used drug, after caffeine from coffee and tea.

The main problem with tobacco smoking is its enormous implications on health. Today it is estimated that smoking-related diseases cause some 3 - 4 million deaths per year. In the US Surgeon General's 1988 report on The Health Consequences of Smoking, it was estimated that in the US alone about 300.000 deaths are caused each year by diseases related to cigarette smoking. In fact, excessive smoking is now recognized as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the world's second most used drug.

The most advantageous thing a heavy smoker can do is to reduce or preferably even stop smoking completely. Experience shows, however, that most smokers find this extremely difficult since, mostly, tobacco smoking results in a dependence disorder or craving. The WHO has in its International Classification of Disorders a diagnosis called Tobacco Dependence. Others, like the American Psychiatric Association call the addiction Nicotine Dependence, It is generally accepted that these difficulties to stop smoking result from the fact that those heavy smokers are dependent on nicotine. The most important risk factors are, however, substances that are formed during the combustion of tobacco, such as carcinogenic tar produce, carbon monoxide, aldehydes, and hydrocyanic acid.

### Effects of nicotine

The administration of nicotine can give satisfaction and the usual method is by smoking, either by smoking e. g. a cigarette, a cigar or a pipe, orby snuffing or chewing tobacco. However, smoking has health hazards and it is therefore desirable to formulate an alternative manner of administering nicotine in a pleasurable manner that can be used to facilitate withdrawal from smoking and/or used as a replacement for smoking.

Upon smoking of a cigarette, nicotine is quickly absorbed into the smoker's blood and reaches the brain within around ten seconds after inhalation. The quick uptake of nicotine gives the consumer a rapid satisfaction, or kick The satisfaction, then, lasts during the time of smoking the cigarette and for a period of time thereafter. The poisonous, toxic, carcinogenic, and addictive nature of smoking has provided efforts for methods, compositions and devices, which help in breaking the habit of smoking.

Nicotine is an addictive poisonous alkaloid C₅H₄NC₄H₇NCH₃, derived from the tobacco plant. Nicotine is also used as an insecticide. Approximately forty milligrams of nicotine may kill an adult (Merck Index).

### Nicotine replacement products and prior art

One way to reduce smoking is to provide nicotine in a form or manner other than by smoking and some products have been developed to fulfill this need. Nicotine containing formulations are currently the dominating treatments for tobacco dependence.

The success in achieving reduction in the incidence of smoking has been relatively poor using presently known products. State of the art involves both behavioral approaches and pharmacological approaches. More than 80% of the tobacco smokers who initially quit smoking after using some behavioral or pharmacological approach to singly reduce smoking incidence generally relapse and return to the habit of smoking at their former rate of smoking within about a one year's period of time.

As an aid for those who are willing to stop smoking there are several ways and forms of nicotine replacement products available on the market, such as nicotine chewing gums according to US 3,845,217. Several methods and means have been described for diminishing the desire of a subject to use tobacco, which comprises the step of administering to the subject nicotine or a derivative thereof as described in e g US 5,939,100 (nicotine containing microspheres) and US 4,967,773 (nicotine containing lozenge).

The use of skin patches for transdermal administration of nicotine has been reported (Rose, in Pharmacological Treatment of Tobacco Dependence, (1986) pp. 158 - 166, Harvard Univ. Press). Nicotine-containing skin patches that are in wide use today can cause local irritation and the absorption of nicotine is slow and affected by cutaneous blood flow.

Nicotine-containing nose drops have been reported (Russell et al., British Medical Journal, Vol. 286, p. 683 (1983); Jarvis et al., British Journal of Addiction, Vol. 82, p. 983 (1987)). Nose drops, however, are difficult to administer and are not convenient for use at work or in other public situations. Administration of nicotine by way of delivery directly into the nasal cavity by spraying is known from US 4,579,858, DE 32 41 437 and US 5,656,255. There may, though, be local nasal irritation with use of nasal nicotine formulations. The difficulty in administration also results in unpredictability of the dose of nicotine administered.

Mouth sprays comprising nicotine are known in the art, e g according to US 6,024,097 wherein is disclosed a method of assisting a smoker in giving up the smoking habit whereby is used a plurality of aerosol dispensers comprising progressively lesser concentrations of nicotine. The aerosol is intended to be administered into the month. The liquid in the dispensers essentially consists of nicotine and alcohol. GB 2 030 862 discloses a nicotine-containing aerosol for oral administration.

A similar mouth spray is disclosed in US 5,810,018, whereby in addition the aerosol comprises progressively greater concentrations of at least one selected stimulant.

US 6,413,496 discloses an aerosol device with an active and a propellant. The device may be used for e g sublingual administration. Nicotine is mentioned as an active in a long "laundry list" of drugs. There are though no examples on nicotine formulations.

US 5,955,098 discloses a non-polar buccal aerosol spray using a non-polar solvent. Nicotine is mentioned as one useful active in this spray.

Inhaling devices resembling a cigarette are known for uptake of nicotine vapors mainly buccally is suggested in US 6,167,242. A proposal on a nicotine aerosol is disclosed in DE 32 41 437. Newman et al, J Pharma Sci, Vol 85, No 9, September 1996 discloses highly ethanolic systems for administering the asthma medication flunisolide as aerosol to the lungs. Possible utility of these systems for delivery of nicotine is though not discussed. Buffering of the medication for facilitating pulmonary delivery is not discussed.

US 4,953,572 and US 4,920,989 disclose one and the same nicotine-containing aerosol intended for inhalation.

US 5,656,255 discloses a method of smoking cessation therapy comprising concurrent transdermal and buccal administration of nicotine, whereby no liquid formulation is disclosed.

WO 03/026655 discloses formulations comprising nicotine and cocoa powder. WO 03/026656 discloses formulations comprising nicotine and chocolate. WO 03/055486 discloses liquid formulations comprising nicotine for administration of nicotine to the oral cavity.

Burch et al disclose in Am Rev Respir Dis 189; 140:955-957 pulmonary inhalation of nicotine in conjunction with buccal deposition of a nicotine formulation having pH 10.

Andrus et al disclose in Can Respir J Vol 6 No 6:509-512 a nicotine microaerosol inhaler wherein nicotine is present in a non-pH regulated formulation comprising ethanol and a propellant.

Hitherto is though not known any pharmaceutical formulations or systems comprising at least 50 % ethanol that efficiently may administer nicotine for uptake mainly in the lungs in order to mimic the nicotine uptake provided by smoking without the adverse effects caused by smoking.

### Problems to be solved

The captioned means and methods do not satisfy the craving that certain users of tobacco experience. Specifically these means and methods generally do not provide for a sufficiently rapid uptake of nicotine without adverse effects. In nicotine replacement therapy, NRT, many smokers wish to obtain a head rush or very quick onset of nicotine similar to the one obtained by inhaling cigarette smoke. None of hitherto known NRT means may provide for this.

In light of the aforementioned problem there is a strong need and interest to develop formulations, for the administration of nicotine to provide a very fast satisfaction to a person craving for nicotine or to provide a sense of smoking satisfaction without smoking, whereby also may be avoided problems associated with the prior art means and methods. The present invention addresses said need and interest.

### Summary of the Invention

In view of the foregoing disadvantages known in the art when trying to deliver nicotine to a subject so as to obtain a rapid uptake of nicotine the present invention provides a new and improved product, for obtaining a rapid uptake of nicotine essentially in the lungs of the subject.

In contrast to smoking, which involves rapid absorption of nicotine, the use of current nicotine replacement Therapy (NRT), achieved through use of nicotine formulated in chewing gum, transdermal patch, tablet, nasal spray and inhaler generally provides slower, lower and less variable plasma nicotine concentrations. The arteriovenous differences during cigarette smoking are substantial, with arterial levels exceeding the venous levels six- to ten-fold. The instant relief from craving during cigarette smoking is believed to be achieved by the "nicotine buzz", the sharp rise of nicotine concentration in arterial blood, the arterial blood being transported from the lung alveoli via the heart to the brain within seconds. The nicotine level in the brain declines between cigarettes as the nicotine is distributed to other body tissues. This decline in nicotine level provides an opportunity for resensitization of the nicotine receptors in brain, allowing some positive reinforcement despite the development of acute tolerance. The current NRT options do not provide this initial sharp rise in arterial blood level. Even if the success rates for smoking cessation with current NRT are twice those obtained using placebo, such therapy is still not very successful. Only up to 30 percent of all people succeed in their attempts to quit smoking. Therefore, there is a strong need for a more efficient therapy for smoking cessation. This invention provides cigarette-like delivery of nicotine.

Objects of the present invention are to provide an efficient and effective product, for an essentially pulmonary uptake of nicotine in a subject to avoid the disadvantages of previously known products and methods.

According to the present invention there is provided a liquid pharmaceutical formulation comprising nicotine in any form characterized in that it is for administration to the lungs, in that it is acidified and/or alkalized by buffering and/or pH-regulation and in that it comprises at least 50%, preferably at least 90% and most preferably 99% ethanol.

Thus, the present invention provides a product for delivering nicotine in any form to a subject by administration essentially into the lungs of the subject. The nicotine in any form is absorbed into the systemic circulation of the subject essentially by pulmonary uptake of nicotine.

The present invention provides for a flexible, convenient and discrete use in comparison with other means for transmucosal delivery of nicotine, e g chewing gums, lozenges, tablets, mouth sprays and other devices attempting to provide nicotine inhalation. No chewing or sucking is necessary. Further, the present liquid pharmaceutical formulation provides nicotine in a form being directly absorbable by a subject. The nicotine in chewing gums, lozenges and tablets need pass a transformation phase, involving e g mastication, disintegration, melting and/or dissolution, prior to being present in a directly absorbable form. A nicotine patch provides for a discrete administration, but does not provide for a fast uptake of nicotine. Also, the formulations of the present invention directly mimic the way a smoker receives nicotine and provide for a head rush and very quick onset of nicotine essentially being the same as experienced with smoking. Therefore a smoker very easily may adapt to using the present invention.

Preferred embodiments of a product according to the present invention is buffered and/or pH regulated in such a way that the pH of the liquid pharmaceutical formulation is from pH 3 to pH 7, preferably from pH 4 to pH6.

Use of said product according to the invention will rapidly deliver nicotine in any form to a subject and will also provide for obtaining a quick and/or sustained and/or complete reduction of the urge to smoke or use tobacco and/or for providing a sense of smoking satisfaction without smoking resembling the sense of smoking satisfaction chained after regular smoking or use of tobacco.

The present liquid formulation is an ethanol-based formulation.

### The buffering agent and the pH regulating means

In a preferred embodiment of the present invention, the composition of the liquid has its pH adjusted to between 3 and 7, Thereby the pH is close to the physiological pH without causing significant irritation. As the pKₐ for the acid dissociation constant of the mono-protonated form of nicotine in an aqueous system is approximately 8.0 at 20° C, at pH's in the range 5 to 7 virtually all of the nicotine (90% to 99.9%) exists in its mono-protonated form and is thereby prevented from existing in the vapor phase. For the ethanol-based system the apparent pK₄ is approximately 6.5 at 20° C and, consequently, at apparent pHs in the range 3 to 5.5 virtually all of the nicotine (90% to 99%) exists in its mono-protonated form and is thereby prevented from existing in the vapor phase.

Hence and according to the invention, the liquid pharmaceutical formulation is acidified and/or alkalized by buffering and/or pH regulation. This may be achieved by including physiologically acceptable buffering substances or agents, or by other means. With other means it is intended to include buffering by any component in the product, which may not normally act as a buffering agent, such as a self-buffering additive and/or pH regulating forms of nicotine.

By buffering and/or pH regulation thereby decreasing the pH of formulation uplake of nicotine takes place mainly in the lungs rather than in the oral cavity or in the respiratory tract. Hence, only small amounts of nicotine will be swallowed and reach the gastrointestinal (GI) tract. Nicotine that reaches the GI tract will be subjected to first pasts metabolism, which reduces the total amount of intact nicotine absorbed. This means that the bioavailability of nicotine that is not co-administered with a buffer according to the invention will generally be lower than when administered together with a buffer.

A titration curve for nicotine with sulphuric acid in a 90:10 ethanol:water matrix shows that the apparent pKa of nicotine in 90% ethanol is 6.5. This means that at apparent pH 6.5, 50% of the nicotine is in its free base form. Separately, an Andersen Cascade Impactor (ACI) experiment, performed with a formulation at an apparent pH = 4.0, demonstrated that nicotine (in its salt form at this apparent pH) remains in the liquid droplets. The majority of the droplets has sizes less than 3.3 microns and would be deposited in the lungs upon inhalation. By comparison, an ACI experiment, performed with a formulation at an apparent pH = 6.0, demonstrated that part of the nicotine (approximately 15% of it is in its free base form at this apparent pH) escapes from the liquid droplets as a vapor. It is well known that inhaled nicotine vapor deposits in the oral cavity. Therefore, the nicotine in its base form, inhaled in this formulation would not be deposited in the longs,

In conclusion, the above set of experiments demonstrates the utility of the present invention for delivering nicotine to the lungs.

An acidifying effect may be achieved by the use of one or more buffering agents selected from the group consisting of citric acid, phosphoric acid, acetic acid, hydrochloric acid, nitric acid, sulphuric acid and acidic salts thereof.

An alkalizing effect may be achieved by the use of one or more buffering agents selected from the group consisting of a carbonate, such as mono-carbonate, bicarbonate or sesquicarbonate; glycinate, phosphate, glycerophosphate, acetate, gluconate or citrate of an alkali metal, such as potassium or sodium, or of ammonium, and mixtures thereof; and/or by the use of pH regulating agents, such as agents selected from the group consisting of sodium hydroxide, potassium hydroxide, calcium hydroxide and calcium oxide; and/or by using at least partly pH regulating forms of nicotine.

The pH regulation may also be obtained by using pH-regulating forms of nicotine, e g nicotine free base.

The nicotine may be administered in different forms, e g in different complexes or salts or as free base.

### Detailed Description of the Invention

### Definitions

The terms "tobacco,", "tobacco containing material" and similar are herein intended to mean such material for any type of use of tobacco. including smoking, snuffing or chewing whereby is used inter alia a cigarette, a cigar, pipe tobacco, snuff and chewing tobacco.

The term "fast reduction of the urge to smoke or use tobacco " is herein intended to mean an initial priming of the subject so as to achieve a reduction of the urge to smoke or use tobacco.

The term "buccal" and "buccally" are herein intended to pertain to all of or any part of the soft tissue lining of the oral cavity.

The term "pulmonary uptake" is herein intended to mean that the active agent passes the lung tissue whereupon it enters the systemic circulation.

The term "delivery to the lungs" and similar is herein intended to mean deposition of an active agent on lung tissue.

The term "incidence of administration" is herein intended to mean administration of one or more single doses of the liquid pharmaceutical formulation within the same time frame, said time frame being dependent on the needs of the subject receiving the administration, said time frame extending from a few seconds to around ten minutes.

### The active ingredient

According to the invention, the liquid pharmaceutical formulation product comprises nicotine in any form to provide an essentially pulmonary uptake of the nicotine so as to obtain a rapid "nicotine kick or buzz" or "nicotine head rush" or reduction of the urge to smoke and/or use tobacco. Thereby may also be achieved a systemic maintenance level of nicotine.

The nicotine should be in a form facilitating the uptake of the nicotine in the lungs.

The nicotine may act as a stimulant to e g obtain a rapid reduction of the urge to smoke or to use tobacco.

With nicotine, it is intended to include nicotine, 3-(1-methyl-2-pyrrolidinyl)-pyridine, including the racemate and the enantiomers, with its base form, including synthetic nicotine as well as nicotine, extracts from tobacco plants, or parts thereof, such as the genus Nicotiana alone or in combination; or pharmaceutically acceptable salts.

In preferred embodiments, the nicotine in any form is selected from the group consisting of the free base form of nicotine, a nicotine salt and/or a nicotine derivative.

Preferred nicotine salts arc salts with the following acids Formic, Acetic, Propionic, Butyric, 2-Methylhutyric, 3-Methylbutyric, Valeric, Lauric, Palmitic, Tartaric, Citric, Malic, Oxalic, Benzoic, Gentisic, Gallic, Phenylacetic, Salicylic, Phthalic, Picric, Sulfosalicylic, Tannic, Pectic, Alginic, Hydrochloric, Chloroplatinic, Silicotungstic, Pyruvic, Glutamic and/or Aspartic.

Most preferred nicotine salts are tartrate, hydrogen tartrate, citrate and malate.

The most preferable embodiment incorporates nicotine as the free base form or as a water-soluble pharmaceutically acceptable salt.

According to the invention, the uptake of the nicotine in the lungs is improved in relation to the lung uptake obtained by pulmonary administration of a theoretical liquid pharmaceutical formulation devoid of buffering agents or devoid of pH-regulating means.

### Amount of the nicotine in the liquid pharmaceutical formulation

The nicotine in any form is according to the invention formulated to provide the subject with a dose to achieve an effect. The effect may be to provide a sense of smoking satisfaction without smoking. Another effect of the administered nicotine in any form may be a reduction of the urge to smoke or use tobacco.

The effect may also be a combination of a redaction of said urge and providing a sense of smoking satisfaction without smoking. The amount of the nicotine should be sufficient to provide such an effect in a subject. This amount may, of course, vary from person to person.

According to the invention, embodiments of the liquid pharmaceutical formulation comprise nicotine in such concentration that the amount of nicotine delivered at each incidence of administration is about 0.05 - 10 mg calculated as the free base form of nicotine, preferably about 0,5 - 5 mg and most preferably about 0.5 - 1 mg.

### Release and uptake of nicotine

The time point for reaching a sense of satisfaction or reduction of urge to smoke or use tobacco after administration is individual, but may in existing pharmaceutical forms for administering nicotine generally be reached after approximately 30 minutes when regarded as coinciding with tₘₐₓ. According to the present invention, such a sense of satisfaction may be reached after a shorter period of time due to a rapid transmucosal uptake in the lungs due to the buffering and/or pH regulation and due to the absence of rate-limiting steps, such as tablet or lozenge melting, tablet or lozenge disintegration and dissolution and chewing gum mastication, followed by drug dissolution.

### The liquid phase

The liquid phase of the present liquid pharmaceutical formulation may comprise water. The liquid phase may also comprise glycerol, propylene glycol and polyethylene glycol, or mixtures thereof. Further it may comprise mixtures of the above ingredients.

One or more of the compounds of the liquid pharmaceutical formulation may be solubilized in one or more surface active agents and/or emulsifiers, such as nonionic, cationic, anionic or zwitterionic surfactants, including amphiphilic block copolymers, or mixtures thereof.

### Other additives to the liquid pharmaceutical formulation

Other additives may be added optionally to the liquid pharmaceutical formulation. These include tonicity agents, preferably chosen from sugars and inorganic salts.

A suitable such formulation may comprise nicotine in any form, water, ethanol and sodium chloride and/or sodium hydroxide and/or hydrochloric acid and optionally one or more tonicity agents, which are preferably chosen form sugars and inorganic salts.

The administration to the lungs preferably takes place by aerosolization.

Aerosolization is preferably achieved by use of a nebulizer. For conventional nebulizers, the nicotine is dissolved in a solution, see above, and the drug solution, is placed in the nebulizer cup. A high velocity air stream is passed over a capillary tube extending into the drug solution. The low pressure created by this jet stream draws the liquid into the jet stream. Internal baffling creates a standing aerosol cloud from which the subject receives the dose upon inhalation. The remainder of the aerosol is recycled within the nebulizer.

Recently, portable nebulizers, oftentimes referred to as Air Mist Inhalers or AMIs, have emerged. These devices generate the standing aerosol cloud by various techniques such as ultrasonic methods, mechanical break-up and electrohydrodynamics. These AMIs overcome most of the drawbacks of conventional nebulizers (bulky size, need for an external power source, low efficiency, long treatment times, etc.). One such AMI is disclosed in Am Rev Respir Dis 1989; 140: 933-937. Anyhow, more efficient AMIs arc envisageable.

The liquid pharmaceutical formulation may be used for obtaining a quick and/or sustained and/or complete reduction of the urge to smoke or use tobacco and/or for providing a sense of smoking satisfaction without smoking as further discussed below.

The fast relief provides the subject with a sense of rapid smoking satisfaction without smoking.

### Cessation of the urge to smoke or use tobacco

For some of the users, it may be a goal to terminate the usage of nicotine completely, due to several reasons c g health, economical, social or behavioral. This may be achieved by further decreasing the delivered amount of nicotine in any form gradually over time. In specific embodiments of the invention, the method described above for obtaining craving relief may further comprise the steps of decreasing the amount of nicotine in the liquid pharmaceutical formulation gradually over time, and/or the steps of educing the incidence of administration of the liquid pharmaceutical formulation gradually over time, and/or the steps of reducing the dosage size of the liquid pharmaceutical formulation gradually over time, so as to achieve a relief of tobacco craving and/or to achieve a sense of smoking satisfaction. This method results in a weaning process gradually over time.

Different types of smokers reach the sense of reduced craving at different plasma levels of nicotine. This may, of course, affect the individual types of programs for administering a liquid pharmaceutical formulation according to the invention, Different types of smokers include e g peak seekers or smokers that crave for a plasma level of nicotine constantly being above the level below which withdrawal symptoms occur.

One strategy may be to lower the frequency of administering the liquid pharmaceutical formulation. Other embodiments include varying the dose of the nicotine in said liquid pharmaceutical formulation as well as the combination of these two embodiments.

### Use of the liquid pharmaceutical formulation

The use of she liquid pharmaceutical formulation according to the invention is for obtaining a fast and/or sustained and/or complete reduction of the urge to smoke and use tobacco or for providing a sense of smoking without smoking as described above.

The douse of the nicotine is chosen to give the subject an individual sensory perception and satisfaction with an effect of the nicotine in any form.

### Examples on embodiments and manufacturing of the liquid pharmaceutical formulation

The below examples are non-limiting and for illustrating the present invention. Alternatives and variations of the below examples within the scope of the present invention as per the below claims may be carried out by a person skilled in the art. Ingredients as per the below examples may be exchanged for equivalent ingredients, preferably as per above.

The present liquid pharmaceutical formulation is an ethanol-based formulation in principle being manufactured as follows:
- Take the required amount of ethanol.
- Add the required amount of water.
- Add the required amount of propylene glycol and/or glycerol.
- Add the required amount of organic acid and/or inorganic acid.
- Mix the ingredients until homogeneous.
- Add the required amount of nicotine as free base or nicotine in salt form.
- Optionally add further ingredients.
- Mix the ingredients until homogeneous.
- Adjust apparent pH, targeting 3.0 to 5.5.

All operations may be done at room temperature and no other ingredients, such as preservatives, are required.

The present liquid pharmaceutical formulation is not limited to the above embodiments or to the below Examples.

### Example 1

Manufacturing of 100 ml ethanol-bascd formulation with 10 µg nicotine/µl and pH around 3.0.
60 g 99% ethanol and 9.9 g (polyethylene) propylene glycol was thoroughly mixed with 1000 mg nicotine at room temperature. Thereafter pH was adjusted to 3.0 using diluted sulphuric acid. Thereafter the volume was adjusted to 100.0 ml with addition of 99% ethanol. The solution was aseptically filtered and put into appropriate sterile container.

### Example 2

Manufacturing of 100 ml ethanol-based formulation with 10 µg nicotine/µl and pH around 4.0.
60 g 99% ethanol and 9.9 g (polyethylene) propylene glycol was thoroughly mixed with 1000 mg nicotine at room temperature. Thereafter pH was adjusted to 4.0 using diluted HCl. Thereafter the volume was adjusted to 100.0 ml with addition of 99% ethanol. The solution was aseptically filtered and put into appropriate sterile container.

### Example 3

Manufacturing of 100 ml ethanol-based formulation with 50 µg nicotine/µl and pH around 5.0.
60 g 99% ethanol and 9.9 g (polyethylene) propylene glycol was thoroughly mixed with 5000 mg nicotine at room temperature. Thereafter pH was adjusted to 5.0 using diluted sulphuric acid. Thereafter the volume was adjusted to 100.0 ml with addition of 99% ethanol. The solution was aseptically filtered and put into appropriate sterile container.

### Example 4

Manufacturing of 100 ml ethanol-based formulation with 50 µg nicotine/µl and pH around 5,5.
60 g 99% ethanol and 9.9 g glycerol was thoroughly mixed with 5000 mg nicotine at room temperature. Thereafter pH was adjusted to 5.5 using diluted sulphuric acid. Thereafter the volume was adjusted to 100.0 ml with addition of 99% ethanol. The solution was aseptically filtered and put into appropriate sterile container.

The liquid pharmaceutical formulation may within the inventive concept comprise ingredients in other amounts than in the above examples.

### Use for therapy, treatment and manufacturing

The liquid pharmaceutical formulation product according to the invention may be used in therapy. Said therapy may be a treatment of a disease or medical indication selected from the group consisting of reduction in use of tobacco, cessation of use of tobacco, other use of tobacco, temporary abstinence from abstaining from use of tobacco, Alzheimer's disease, Crohn's disease, Parkinson's disease, Tourette's syndrome, and ulcerative colitis; and weight control.

Nicotine in any form may be used for the manufacturing of a liquid pharmaceutical formulation according to the invention for the treatment of a disease or medical indication selected from the group consisting of reduction in use of tobacco, cessation of use of tobacco, other use of tobacco, temporary abstinence from abstaining from using tobacco, Alzheimer's disease, Crohn's disease, Parkinson's disease, Tourette's syndrome, and ulcerative colitis; and weight control.

## Claims

1. A liquid pharmaceutical formulation comprising nicotine in any form, **characterzed in that** it is for administration to the lungs, in that it is acidified and/or alkalized by buffering and/or pH regulation, and in that it comprises at least 50 %, preferably at least 90 % aud most preferably 99 % ethanol.

2. A liquid pharmaceutical formulation according to claim 1, **charactarized in that** it is for administration to the lungs as an aerosol.

3. A liquid pharmaceutical formulation according to any of claims 1 or 2, **characterized in that** it is acidified and/or alkalized by buffering and/or pH regulation in such a way that a pH from, is obtained in the formulation,

4. A liquid pharmaceutical formulation according to any of claims 1 - 3, **characterized in that** its pH is adjusted and/or regulated by use of one or more buffering agents selected from the group censing of citric acid, phosphoric acid, acetic acid, hydrochloric acid, nitric acid and acidic salts thereof, and/or from the group consisting of a carbonate, such as mono-carbonate, bicarbonate or sesquicarbonate; glycinate, phosphate, glycerophosphate, acetate, gluconate or citrate of an alkali metal, such as potassium or sodium, or of ammonium, and mixtures thereof, and/or by use of pH regulating agents, such as agents selected from the group consisting of sodium hydroxide, potassium hydroxide, calcium hydroxide and calcium oxide; and/or by using at least partly pH regulating forms of nicotine.

5. A liquid pharmaceutical formulation according to any of claims 1 - 3, **characterized in that** it is acidified by buffering and/or pH regulation by the use of one or more buffering agents selected from the group consisting of citric acid, phosphoric acid, acetic acid, hydrochloric acid, nitric acid and acidic salts thereof, and/or by using at least partly pH regulating forms of nicotine.

6. A liquid pharmaceutical formulation according to any of claims 1 - 5, wherein the nicotine in any form is selected from the group consisting of the free base form of nicotine, a nicotine salt, and/or a nicotine derivative.

7. A liquid pharmaceutical formulation according to claim 6, wherein the nicotine salt is a salt formed as tartrate, hydrogen tartrate, citrate or malate.

8. A liquid pharmaceutical formulation according to any of claims 1 - 7, wherein the amount of nicotine in any form delivered at each incidence of administration is about 0.05 - 10 mg calculated as the free base form of nicotine.

9. A liquid pharmaceutical formulation according to claim 8, wherein the amount of nicotine in any form delivered at each incidence of administration is about 0.5 - 5 mg calculated as the free base form of nicotine.

10. A liquid pharmaceutical formulation according to claim 9, wherein the amount of nicotine in any form delivered at each incidence of administration is about 0.5 - 1 mg calculated as the free base form of nicotine.

11. A liquid pharmaceutical formulation according to any of claims 1 - 10, which further comprises water.

12. A liquid pharmaceutical formulation according to any of claims 1 - 11, which further comprises glycerol, propylene glycol or polyethylene glycol, or mixtures thereof.

13. A liquid pharmaceutical formulation according to any of claims 1 - 12, **characterized in that** one or more of the compounds of the liquid pharmaceutical formulation is/are solubilized in one or more surface active agents and/or emulsifiers, such as nonionic, cationic, anionic or zwitterionic surfactants, including amphiphilic block copolymers, or mixtures thereof.

14. A liquid pharmaceutical formulation according to any of claims 1 - 13, **characterized in that** it comprises nicotine in any form, water, ethanol and sodium chloride and/or sodium hydroxide and/or hydrochloric acid and optionally one or more tonicity agents.

15. A liquid pharmaceutical formulation according to claim 14, **characterized in that** the optionally one or more tonicity agents are chosen from sugars and inorganic salts.

16. A formulation according to any of claims 1 - 15 for use in therapy.

17. A liquid pharmaceutical formulation according to claim 16, wherein the therapy is treatment of a disease selected from the group consisting of addiction to tobacco or nicotine, Alzheimer's disease, Crohn's disease, Parkinson's disease, Tourette's syndrome, ulcerative colitis; and weight control.

18. Use of nicotine in any form for the manufacturing of a liquid pharmaceutical formulation according to any of claims 1 - 15 for the treatment of a disease selected from the group consisting of addiction to tobacco or nicotine, Alzheimer's disease, Crohn's disease, Parkinson's disease, Tourette's syndrome, ulcerative colitis; and weight control.

## Patentansprüche

1. Flüssige pharmazeutische Formulierung, die Nikotin in einer beliebigen Form umfasst, **dadurch gekennzeichnet, dass** sie an die Lungen zu verabreichen ist, dass sie durch Pufferung und/oder pH-Wert-Regulierung angesäuert und/oder alkalisiert ist, und dass sie mindestens 50 %, vorzugsweise 90 % und am meisten bevorzugt 99 % Ethanol umfasst.

2. Flüssige pharmazeutische Formulierung gemäß Anspruch 1, **dadurch gekennzeichnet, dass** sie als Aerosol an die Lungen zu verabreichen ist.

3. Flüssige pharmazeutische Formulierung gemäß einem der Ansprüche 1 oder 2, **dadurch gekennzeichnet, dass** sie derart durch Pufferung und/oder pH-Wert-Regulierung angesäuert und/oder alkalisiert ist, dass ein pH-Wert von 3 bis 7, vorzugsweise von 4 bis 6 in der Formulierung erhalten wird.

4. Flüssige pharmazeutische Formulierung gemäß einem der Ansprüche 1 - 3, **dadurch gekennzeichnet, dass** ihr pH-Wert durch Verwendung von einer oder mehreren puffernden Substanzen eingestellt und/oder reguliert wird, die ausgewählt sind aus der Gruppe bestehend aus Zitronensäure, Phosphorsäure, Essigsäure, Salzsäure, Salpetersäure und deren sauren Salzen, und/oder aus der Gruppe bestehend aus einem Carbonat, wie etwa Monocarbonat, Bicarbonat (Hydrogencarbonat) oder Sesquicarbonat; Glycinat, Phosphat, Glycerophosphat, Acetat, Gluconat oder Citrat eines Alkalimetalls, wie etwa Kalium oder Natrium, oder von Ammonium, und deren Gemischen; und/oder durch Verwendung von pH-Wert-Regulatoren, wie etwa Regulatoren, die ausgewählt sind aus der Gruppe bestehend aus Natriumhydroxid, Kaliumhydroxid, Calciumhydroxid und Calciumoxid; und/oder durch zumindest partielle Verwendung von pH-Wert-regulierenden Formen von Nikotin.

5. Flüssige pharmazeutische Formulierung gemäß einem der Ansprüche 1 - 3, **dadurch gekennzeichnet, dass** sie durch Pufferung und/oder pH-Wert-Regulierung durch Verwendung von einer oder mehreren puffernden Substanzen angesäuert ist, die ausgewählt sind aus der Gruppe bestehend aus Zitronensäure, Phosphorsäure, Essigsäure, Salzsäure, Salpetersäure und deren sauren Salzen, und/oder durch zumindest partielle Verwendung von pH-Wert-regulierenden Formen von Nikotin.

6. Flüssige pharmazeutische Formulierung gemäß einem der Ansprüche 1 - 5, wobei das Nikotin in einer beliebigen Form ausgewählt ist aus der Gruppe bestehend aus der freien Base von Nikotin, einem Nikotinsalz und/oder einem Nikotinderivat.

7. Flüssige pharmazeutische Formulierung gemäß Anspruch 6, wobei das Nikotinsalz ein Salz ist, das als Tartrat, Hydrogentartrat, Citrat oder Malat ausgebildet ist.

8. Flüssige pharmazeutische Formulierung gemäß einem der Ansprüche 1 - 7, wobei die Menge an Nikotin in einer beliebigen Form, die bei jedem Verabreichungsereignis abgegeben wird, etwa 0,05 - 10 mg beträgt, berechnet als freie Base von Nikotin.

9. Flüssige pharmazeutische Formulierung gemäß Anspruch 8, wobei die Menge an Nikotin in einer beliebigen Form, die bei jedem Verabreichungsereignis abgegeben wird, etwa 0,5 - 5 mg beträgt, berechnet als freie Base von Nikotin.

10. Flüssige pharmazeutische Formulierung gemäß Anspruch 9, wobei die Menge an Nikotin in einer beliebigen Form, die bei jedem Verabreichungsereignis abgegeben wird, etwa 0,5 - 1 mg beträgt, berechnet als freie Base von Nikotin.

11. Flüssige pharmazeutische Formulierung gemäß einem der Ansprüche 1 - 10, die außerdem Wasser umfasst.

12. Flüssige pharmazeutische Formulierung gemäß einem der Ansprüche 1 - 11, die außerdem Glycerin, Propylenglykol oder Polyethylenglykol oder deren Gemische umfasst.

13. Flüssige pharmazeutische Formulierung gemäß einem der Ansprüche 1 - 12, **dadurch gekennzeichnet, dass** eine oder mehrere der Verbindungen der flüssigen pharmazeutische Formulierung in einer/einem oder mehreren oberflächenaktiven Substanzen und/oder Emulgatoren solubilisiert ist/sind, wie etwa nicht-ionischen, kationischen, anionischen oder zwitterionischen Tensiden, einschließlich amphiphilen Block-Copolymeren oder deren Gemischen.

14. Flüssige pharmazeutische Formulierung gemäß einem der Ansprüche 1 - 13, **dadurch gekennzeichnet, dass** sie Nikotin in einer beliebigen Form, Wasser, Ethanol und Natriumchlorid und/oder Natriumhydroxid und/oder Salzsäure und wahlweise ein oder mehrere die Tonizität beeinflussende Mittel umfasst.

15. Flüssige pharmazeutische Formulierung gemäß Anspruch 14, **dadurch gekennzeichnet, dass** das/die wahlweise ein oder mehreren die Tonizität beeinflussenden Mittel ausgewählt sind aus Zuckern und anorganischen Salzen.

16. Formulierung gemäß einem der Ansprüche 1 - 15 zur therapeutischen Verwendung.

17. Flüssige pharmazeutische Formulierung gemäß Anspruch 16, wobei die therapeutische Verwendung einer Behandlung einer Erkrankung dient, die ausgewählt ist aus der Gruppe bestehend aus Tabak- oder Nikotinabhängigkeit, Alzheimer-Erkrankung, Morbus Crohn, Parkinson-Erkrankung, Tourette-Syndrom, Colitis ulcerosa; und Gewichtskontrolle.

18. Verwendung von Nikotin in einer beliebigen Form zur Herstellung einer flüssigen pharmazeutischen Formulierung gemäß einem der Ansprüche 1 - 15 zur Behandlung einer Erkrankung, die ausgewählt ist aus der Gruppe bestehend aus Tabak- oder Nikotinabhängigkeit, Alzheimer-Erkrankung, Morbus Crohn, Parkinson-Erkrankung, Tourette-Syndrom, Colitis ulcerosa; und Gewichtskontrolle.

## Revendications

1. Formulation pharmaceutique liquide comprenant de la nicotine sous une forme quelconque, **caractérisée en ce qu'**elle est destinée à être administrée dans les poumons, **en ce qu'**elle est acidifiée et/ou alcalinisée par tamponnage et/ou régulation du pH, et **en ce qu'**elle comprend au moins 50 %, de préférence au moins 90 % et plus préférablement encore 99 % d'éthanol.

2. Formulation pharmaceutique liquide selon la revendication 1, **caractérisée en ce qu'**elle est destinée à être administrée dans les poumons sous forme d'un aérosol.

3. Formulation pharmaceutique liquide selon l'une quelconque des revendications 1 ou 2, **caractérisée en ce qu'**elle est acidifiée et/ou alcalinisée par tamponnage et/ou régulation du pH de façon qu'un pH de 3 à 7, de préférence de 4 à 6, soit obtenu dans la formulation.

4. Formulation pharmaceutique liquide selon l'une quelconque des revendications 1-3, **caractérisée en ce que** son pH est ajusté et/ou régulé par l'utilisation d'un ou de plusieurs agents de tamponnage choisis dans le groupe constitué par l'acide citrique, l'acide phosphorique, l'acide acétique, l'acide chlorhydrique, l'acide nitrique et leurs sels acides, et/ou dans le groupe constitué par un carbonate comme un monocarbonate, un bicarbonate ou un sesquicarbonate; un glycinate, un phosphate, un glycérophosphate, un acétate, un gluconate ou un citrate d'un métal alcalin, comme le potassium ou le sodium, ou d'ammonium, et leurs mélanges, et/ou par l'utilisation d'agents régulateurs du pH, comme des agents choisis dans le groupe constitué par l'hydroxyde de sodium, l'hydroxyde de potassium, l'hydroxyde de calcium et l'oxyde de calcium; et/ou par l'utilisation de formes de nicotine au moins partiellement régulatrices du pH.

5. Formulation pharmaceutique liquide selon l'une quelconque des revendications 1-3, **caractérisée en ce qu'**elle est acidifiée par tamponnage et/ou régulation du pH par l'utilisation d'un ou de plusieurs agents de tamponnage choisis dans le groupe constitué par l'acide citrique, l'acide phosphorique, l'acide acétique, l'acide chlorhydrique, l'acide nitrique et leurs sels acides, et/ou par l'utilisation de formes de nicotine au moins partiellement régulatrices du pH.

6. Formulation pharmaceutique liquide selon l'une quelconque des revendications 1-5, dans laquelle la nicotine sous une forme quelconque est choisie dans le groupe constitué par la forme de base libre de la nicotine, un sel de nicotine et/ou un dérivé de nicotine.

7. Formulation pharmaceutique liquide selon la revendication 6, dans laquelle le sel de nicotine est un sel formé en tant que tartrate, hydrogénotartrate, citrate ou malate.

8. Formulation pharmaceutique liquide selon l'une quelconque des revendications 1-7, dans laquelle la quantité de nicotine sous une forme quelconque délivrée à chaque administration, calculée en termes de base libre de la nicotine, est d'environ 0,05 - 10 mg.

9. Formulation pharmaceutique liquide selon la revendication 8, dans laquelle la quantité de nicotine sous une forme quelconque délivrée à chaque administration, calculée en termes de base libre de la nicotine, est d'environ 0,5 - 5 mg.

10. Formulation pharmaceutique liquide selon la revendication 9, dans laquelle la quantité de nicotine sous une forme quelconque délivrée à chaque administration, calculée en termes de base libre de la nicotine, est d'environ 0,5 - 1 mg.

11. Formulation pharmaceutique liquide selon l'une quelconque des revendications 1-10, comprenant en outre de l'eau.

12. Formulation pharmaceutique liquide selon l'une quelconque des revendications 1-11, comprenant en outre du glycérol, du propylèneglycol ou du polyéthylèneglycol, ou leurs mélanges.

13. Formulation pharmaceutique liquide selon l'une quelconque des revendications 1-12, **caractérisée en ce que** l'un ou plusieurs des composés de la formulation pharmaceutique liquide est/sont solubilisé(s) dans un ou plusieurs agents tensioactifs et/ou émulsifiants, comme des agents tensioactifs non ioniques, cationiques, anioniques ou zwitterioniques, y compris des copolymères à blocs amphiphiles, ou leurs mélanges.

14. Formulation pharmaceutique liquide selon l'une quelconque des revendications 1-13, **caractérisée en ce qu'**elle comprend de la nicotine sous une forme quelconque, de l'eau, de l'éthanol et du chlorure de sodium et/ou de l'hydroxyde de sodium et/ou de l'acide chlorhydrique et éventuellement un ou plusieurs agents de tonicité.

15. Formulation pharmaceutique liquide selon la revendication 14, **caractérisée en ce que** le ou les agents de tonicité éventuels sont choisis parmi des sucres et des sels inorganiques.

16. Formulation selon l'une quelconque des revendications 1-15, destinée à être utilisée en thérapie.

17. Formulation pharmaceutique liquide selon la revendication 16, dans laquelle la thérapie est le traitement d'une maladie choisie dans le groupe constitué par l'addiction au tabac ou à la nicotine, la maladie d'Alzheimer, la maladie de Crohn, la maladie de Parkinson, le syndrome de Tourette, la colite ulcéreuse; et le contrôle du poids.

18. Utilisation de nicotine sous une forme quelconque pour la fabrication d'une formulation pharmaceutique liquide selon l'une quelconque des revendications 1-15 destinée au traitement d'une maladie choisie dans le groupe constitué par l'addiction au tabac ou à la nicotine, la maladie d'Alzheimer, la maladie de Crohn, la maladie de Parkinson, le syndrome de Tourette, la colite ulcéreuse; et au contrôle du poids.
